# EUROPEAN PATENT SPECIFICATION

(11) **EP 2 097 756 B1**
(45) Date of publication and mention of the grant of the patent: **19.10.2011**
(21) Application number: 07865898.6
(22) Date of filing: 20.12.2007
(51) Int. Cl.: G01N 33/68

(54) **ANTIBODY QUANTITATION**
ANTIKÖRPERQUANTIFIZIERUNG
ANALYSE QUANTITATIVE D'ANTICORPS

(30) Priority: 21.12.2006 US 871378 P
(43) Date of publication of application: 09.09.2009
(73) Proprietor: Novartis AG, 4056 Basel (CH)
(72) Inventor: GRANDA, Brian Walter, Salisbury, Massachusetts 01952 (US); WALL, Daniel B., Salem, Massachusetts 01970 (US); WANG, Yingqi Karen, Boxborough, Massachusetts 01719 (US)
(74) Representative: Bourgarel, Denis Jacques Marie
(86) International application number: PCT/US2007/088258
(87) International publication number: WO 2008/079914

(56) References cited:
- WO-A-2005/101017
- JONES R G A ET AL: "Enhanced pepsin digestion: a novel process for purifying antibody F(ab')2 fragments in high yield from serum" JOURNAL OF IMMUNOLOGICAL METHODS, ELSEVIER, AMSTERDAM, NL, vol. 263, no. 1-2, 1 May 2002 (2002-05-01), pages 57-74, XP004354385 ISSN: 0022-1759
- BOUSHABA RIHAB ET AL: "Kinetics of whole serum and prepurified IgG digestion by pepsin for F(ab')2 manufacture." BIOTECHNOLOGY PROGRESS, vol. 19, no. 4, July 2003 (2003-07), pages 1176-1182, XP002476468 ISSN: 8756-7938
- ANDERSON LEIGH ET AL: "Quantitative mass spectrometric multiple reaction monitoring assays for major plasma proteins" MOLECULAR & CELLULAR PROTEOMICS, ASBBM, BIRMINGHAM, AL, US, vol. 5, no. 4, April 2006 (2006-04), pages 573-588, XP002438814 ISSN: 1535-9476

## Description

The invention relates to a novel method for determining the amount of an antibody of interest in a biological sample. In one embodiment, the method of the invention is characterized in that it comprises at least a step of protein depletion of said biological sample by pepsin digestion to produce F(ab)₂ fragments. In a preferred embodiment, one or more peptides identifying the sequence of the antibody of interest is monitored using high-performance liquid chromatography with tandem mass spectrometry detection (HPLC-MS/MS).

### BACKGROUND

The ability to accurately and reliably determine the pharmacokinetics of pharmaceutical compounds in a variety of preclinical models and clinical samples is essential to designing dosing regimes for optimal efficacy and minimal toxicity. This maximizes the chances for success in late-stage clinical trials and is a necessary component in the regulatory approval process. Due to their high likelihood for success and reduced development times/costs, therapeutic monoclonal antibodies (mAbs) are becoming increasingly important to the pharmaceutical industry. These proteins are immunoglobulins (IgGs) which act by binding the target and inhibiting its activity/reducing its concentration. Monitoring mAb concentrations in serum is commonly performed by using enzyme-linked immunosorbent assays (ELISAs). This technique is sensitive (LOQs as low as picograms/mL) and fast enough to analyze thousands of samples. However, it suffers from significant limitations including poor precision (CVs up to 30%) and accuracy. In addition, it may be subject to matrix interferences such as anti-drug antibodies and has a long development time. Furthermore, when switching between preclinical models, from preclinical to clinical samples or dehumanizing the antibody to reduce toxicological effects in the preclinical model, the assay often needs to be redeveloped. As a consequence, it would be useful to develop other methods to improve the confidence in the data and reduce development times/expenses.

High-performance liquid chromatography with tandem mass spectrometry detection (HPLC-MS/MS) is a widely-used method for quantitating small molecule pharmaceuticals from biological matrices. A triple quadrupole mass spectrometer operating in multiple-reaction monitoring mode (MRM) typically provides the lowest LOQs, highest specificity, best precision and accuracy and highest throughput, although other types of mass spectrometry methods may also be employed. Only recently has this technology been extended to the quantitation of proteins from complex samples such as serum and cell lysates (Gerber et. al. (2003)). This technology is also described for detection of antibody-drug conjugates in PCT patent application W02005/101017 (2005, Genentech). For this application, the sample is generally digested with trypsin and proteotypic peptides are monitored with HPLC-MS/MS and used to calculate the protein concentration. Sample preparation techniques for this analysis typically entail depletion of the most abundant proteins with LOQs in the 1-5 µg/mL range (Anderson and Hunter (2006)). The depletion is accomplished with immunoaffinity columns or dyes and has significant limitations with respect to cost, total serum capacity and throughput. Furthermore, depletion kits may have reduced effectiveness for different species. In addition, with mAbs, the only type of depletion feasible from commercially available sources is albumin. Enrichment of IgGs may be possible using Protein A/G columns or beads, however, this strategy also suffers from similar limitations. Pepsin is a relatively nonspecific protease that has been used by many groups to produce fragments of antibodies which retain the ability to bind antigen (F(ab)₂s) from IgGs (Rousseaux et. al. (1980)). Recently, Jones and Landon (2002 and 2003) demonstrated that pepsin may be used for commercial production of F(ab)₂s from ovine serum samples. F(ab)2 production from whole serum by pepsin digestion has been shown by Boushaba Rihab et al.. Production rates have been measured, but initial antibody concentration was not determined ("Kinetics of whole serum and prepurified IgG digestion by pepsin for F(ab')2 manufacture*. Biotechnology Progress; vol. 19, no.4, July 2003, pages 1176-1182). The present invention derives from the first demonstration that pepsin-like enzymes could be used to deplete serum and enrich for F(ab)₂ as the first step in an assay for quantitation of mAbs from serum, e.g., a mass spectrometry based assay. This approach is cost-effective, universal (independent of species and mAb of interest) and has unlimited capacity. In addition, it depletes peptides associated with the Fc region of the IgGs. These peptides are the most highly conserved and most abundant. Removal of these reduces MS ion suppression and lowers the LLOQ.

### DETAILED DESCRIPTION OF THE INVENTION

The present invention relates to an *in vitro* method for determining the amount of an antibody of interest in a biological sample, said biological sample further comprising proteins other than antibodies, characterized in that said method comprises the steps of:
a) incubating the biological sample with a pepsin-like protease under conditions appropriate for proteolysis of the non-immunoglobulin proteins and digestion of the antibody molecules to produce antibody fragments comprising at least a CDR region; and,
b) determining the amount of the antibody fragments derived from the antibody of interest; said amount of antibody fragments correlating with the amount of antibody of interest in the biological sample.

Unless stated otherwise, the following terms and phrases as used herein are intended to have the following meanings:

The term "antibody", as used herein, refers to full length or one or more fragments of an antibody that retain the ability to specifically bind to an antigen. It has been shown that the antigen-binding function of an antibody can be performed by fragments of a full-length antibody, the "binding fragments". Examples of binding fragments include a Fab fragment, a monovalent fragment consisting of the V_{L}, V_{H}, C_{L} and CH1 domains; a F(ab)₂ fragment, a bivalent fragment comprising two Fab fragments linked by a disulfide bridge at the hinge region; a Fd fragment consisting of the V_{H} and CH1 domains; a Fv fragment consisting of the V_{L} and V_{H} domains of a single arm of an antibody; a dAb fragment (Ward et al., 1989), which consists of a V_{H} domain; and an isolated complementarity determining region (CDR).

Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Huston et al., 1988). Such single chain antibodies are also intended to be encompassed within the term "antigen-binding portion" of an antibody.

The term "monoclonal antibody" as used herein refers to a preparation of antibody molecules of single molecular composition. A monoclonal antibody composition displays a single binding specificity and affinity for a particular epitope.

The term "peptide of interest" refers preferably to a peptide comprised in the antibody fragments generated by pepsin-like digestion that shows experimentally advantageous chromatographic and mass spectrometric properties. In one embodiment, the peptide of interest or a combination or peptides of interest is unique, i.e., it can be found only in the antibody of interest. Advantageous chromatographic performance is defined as narrow peaks with high peptide recovery while good mass spectrometric performance is indicated by relatively high parent ion and fragment ion intensities with a high degree of selectivity for the sequence of the "peptide of interest."

The term "internal standard" refers to a labeled version of the "peptide of interest" wherein the sequence, structure and chemical properties are similar to the "peptide of interest" but the mass is different. For example the "internal standard" could be labeled with heavy isotopes such as 13C, deuterium and/or 15N. Other labeling strategies can be used as long as the aforementioned criteria are met.

The term "antibody of interest" refers to the specific antibody comprised in a biological sample, to be quantitated.

The term "biological sample" means (i) blood, bile, urine, or feces; (ii) tissue extract; and (iii) cell culture media, cell lysate, or cell extract. Preferably, the biological sample is serum from any biological source comprising the antibody of interest.

The term "biological source" means preferably (i) mammalian source such as a mouse, a rat, a rabbit, a dog, a monkey, or a human; and include mammalian tissue; and/or mammalian cultured cells.

The term "quantitating" means determining the relative or absolute quantity of a chemical, such as a peptide or protein, in a biological sample. Absolute quantity is generally determined in terms of concentration ranges.

The present invention derives from the finding that certain proteases can be used to enrich biological sample with antibody fragments comprising at least a CDR region such as F(ab)₂ fragments. The biological sample is thereby depleted from non-immunoglobulin proteins. The remaining stable antibody fragments are then suitable for mass spectrometry based quantitation of the antibody of interest contained in the biological sample.

For ease of reading, proteases capable of digesting non-immunoglobulin proteins, for example non-immunoglobulin proteins present in a serum, but retaining antibody fragments comprising at least a CDR region are referred hereafter as "pepsin-like proteases". Examples of such pepsin-like proteases include, without limitation, pepsin, papain, elastase, ficin, bromelain or V8 protease. Preferred "pepsin-like proteases" are those capable of retaining at least the variable region of antibodies and more preferably F(ab) or F(ab)₂ fragments. The phrase "retaining antibody fragments" means that the antibody fragments are either not degraded by pepsin-like proteases, or are more resistant to proteolysis than the majority of other proteins present in the biological sample under appropriate reaction conditions. The incubation time can range from minutes to days.

In a preferred embodiment, said pepsin-like protease is pepsin as well as any suitable homologues or orthologues thereof, capable of digesting non-immunoglobulin proteins but retaining stable F(ab)₂ fragments. For example, porcine pepsin is commercially available. Suitable homologues or orthologues of pepsin, will share at least 50% amino acid identity, more preferably at least 80% and most preferably at least 90% identity with porcine pepsin when aligned with an optimal alignment. Optimal alignment of sequences for determining a comparison window may be conducted by the local homology algorithm of Smith and Waterman (1981).

In a preferred embodiment, a suitable concentration of the pepsin-like protease, preferably pepsin, for protein depletion of a biological sample in the method of the invention is ranging from 1:100 to 1:10 (wt/wt) enzyme:protein ratios.

In another specific embodiment of the method, step b) comprises the following sub-steps of:
b1) addition of one or more internal standard peptides to the antibody fragments generated in step a) after pepsin digestion has been deactivated;
b2) sample processing, i.e., digesting the sample obtained in step b1) with another protease, preferably, non pepsin-like protease, and optionally, extracting the sample peptides ;
b3) peptide separation to reduce sample complexity, i.e., partially separating the sample peptides from one another by chromatographic and/or other means in one or more samples;
b4) mass spectrometry analysis of the peptides of interest, i.e., measuring by mass spectrometry the mass or mass to charge ratio of the sample peptides present in one or more of the separated samples and identifying peak signals corresponding to peptides of interest from the antibody of interest as well as the internal standard peptides; and,
b5) quantitation of the peptides of interest, e.g., comparing said peak signals to peak signals derived from a standard curve of antibody added at varying known concentrations into blank biological source material using the internal standard for signal normalization for determining the amount of the peptides of interest in the sample, thereby determining the amount of the antibody of interest comprised in the biological sample.

### Sample processing:

The resulting sample obtained in step b1) can be further treated at step b2) with another protease which is not a "pepsin-like" protease. One or more proteases may also be used together, or with other enzymes, to thereby degrade sample components.

For example, the proteolytic enzyme trypsin is a serine protease that cleaves peptide bonds between lysine or arginine and an unspecific amino acid to thereby produce peptides that comprise an amine terminus (N- terminus) and lysine or arginine carboxyl terminal amino acid (C- terminus). In this way the peptides from the cleavage of the protein are specific and predictable and their presence and/or quantity, in a sample from a trypsin digest, can be indicative of the presence and/or quantity of the protein of their origin. Other exemplary proteolytic enzymes include ArgC, LysC, V8 protease and AspN.

For example, F(ab)₂ fragments of the antibody of interest obtained by pepsin digestion might produce three peptides (e.g. peptides A, B and C) when further digested with a protease such as trypsin. Accordingly, a sample that has been digested with a proteolytic enzyme, such as trypsin, and that when analyzed is confirmed to contain peptides A, B and C, can be said to have originally comprised the antibody of interest. If digestion of the F(ab)₂ fragment by trypsin is complete, or adequate controls are in place to account for incomplete digestion, any determination of the identity and/or quantity of one or more of peptides A, B and C in a sample (or a fraction thereof), can be used to identify and/or quantify the antibody of interest contained in the original sample (or a fraction thereof).

Optionally, the sample peptides obtained after sample processing are extracted, using preferably extraction methods appropriate to remove any undesired materials that may affect, decrease sensitivity and reproducibility of the results of mass spectrometry analysis. For example, solid phase extraction can be used (SPE), thereby removing excess salts and detergents, residual serum lipids, non-tryptic peptides resulting from pepsin digestion and insoluble materials and permit significant concentration of the peptides in the sample.

### Peptide separation to reduce sample complexity

In step b3), in order to analyze the sample mixture, the peptides can be partially separated from one another and mass analysis is performed on only a fraction of the sample mixture. In this way, the complexity of the analysis can be substantially reduced since separated peptides can be individually analyzed for mass thereby increasing the sensitivity of the analysis process. Of course the analysis can be repeated one or more times on one or more additional fractions of the sample mixture to thereby allow for the analysis of all fractions of the sample mixture.

Separation conditions under which nearly identical peptide(s) of interest and internal standard peptides that are differentially labeled co-elute or nearly co-elute at a concentration, or in a quantity, that is in proportion to their abundance in the sample mixture can be used to determine the amount of each peptide in each of the samples that comprise the sample mixture. As stated above in section b5, the quantitation of the peptide(s) of interest, and thus the antibody of interest, can be determined by use of internal standard peptide(s) added into each sample after the pepsin has been deactivated. A standard curve of known concentrations of the antibody of interest in blank biological matrix is also generated with fixed amounts of the internal standard peptide(s). The amount of the antibody of interest in any sample from the same or similar matrix, containing the same internal standard, can thus be calculated from the standard curve. A key assumption of this approach is that the spiked antibody in the standard curve will undergo similar sample preparation and digestion efficiency related effects to the same antibody from the test samples. Thus it is reasonable to use the standard curve from the spiked antibody in the blank matrix, along with the internal standard peptide(s), to quantify the antibody from the samples in the study, where the same internal standard is used. In addition the internal standard serves to normalize the MS signal intensities and thus reduce variability from some stages of sample preparation and analysis. Accordingly, in some embodiments, separation of the sample mixture can simplify the analysis whilst maintaining the correlation between signals determined in the mass analysis (e.g. MS/MS analysis) for the internal standard peptide(s) and the peptide(s) of interest in the sample mixture.

In certain embodiments, the separation can be performed by chromatography. Any chromatographic separation process suitable to separate the peptide(s) of interest can be used. The chromatographic separation can be normal phase chromatography, reversed-phase chromatography, ion-exchange chromatography, size exclusion chromatography or affinity chromatography. For example, liquid chromatography/mass spectrometry (LC/MS) can be used to effect such a sample separation and mass analysis. In a preferred embodiment, the separation step b3) is performed by HPLC.

### Mass spectrometry analysis of the peptides of interest

The methods of the invention are appropriate for the analysis of peptides derived from the antibody of interest in the biological samples where the peptide(s) of interest are first isolated, separated, or partially separated by one or more of the separation processes described below. For selective quantitation of the antibody of interest, the peptide(s) of interest preferably comprises at least one portion selected in the variable region of the antibody of interest. The peptide(s) should further be selected from the portion of the amino acid sequence that enables discrimination of the peak signals corresponding specifically to the fragments of the antibody of interest from non specific signal corresponding to other protein fragments, or conserved amino acid sequences among antibodies. The sequences of such peptide(s) of interest can be selected among those which contain unique amino acid residues within the variable light and/or heavy chain regions of an antibody when compared to corresponding native IgGs sequences obtained from a native IgGs sequence database. Ideally, the peptide(s) of interest should also have good ionization and chromatographic properties, no residues highly susceptible to modification (such as methionine) and no detectable background from the total IgG from the species of interest, for example, total human IgG.

Preferably, in step b4), identification of mass or mass to charge ratios of the digested proteins, including the "peptide(s) of interest", in a separated sample can be performed by any mass spectrometers with sufficient selectivity and sensitivity available in the art.

It can be practiced using for example tandem mass spectrometers and other mass spectrometers that have the ability to select and fragment molecular ions. Tandem mass spectrometers (and to a lesser degree single-stage mass spectrometers) have the ability to select and fragment molecular ions according to their mass-to-charge (m/z) ratio, and then record the resulting fragment (daughter) ion spectra. More specifically, daughter fragment ion spectra can be generated by subjecting selected ions to dissociative energy levels (e.g. collision- induced dissociation (CID)). For example, ions corresponding to labeled peptides of a particular m/z ratio can be selected from a first mass analysis, fragmented and reanalyzed in a second mass analysis. Representative instruments that can perform such tandem mass analysis include, but are not limited to, magnetic four-sector, tandem time-of- flight, triple quadrupole, ion-trap, and hybrid quadrupole time-of-flight (Q-TOF) mass spectrometers.

In a preferred embodiment, step b4) is performed by tandem mass spectrometry (MS/MS). Preferably, triple quadrupole for multiple reaction monitoring (MRM) is used for tandem mass analysis. Briefly, in MRM, the first quadrupole is used to isolate the parent mass of the peptide. The parent mass is representative of the overall sequence of the peptide. It then passes into the collision quadrupole where it is fragmented. The third quadrupole is used to monitor selected fragment ions. The fragments generated are indicative of the internal peptide sequence.

### Quantitation of the peptides of interest:

Quantitative measurements of the peptide(s) of interest can be obtained from mass analysis in step b4).

In some embodiments, quantitation of peptide(s) of interest normalized to analogous internal standard peptide(s) may be achieved using calculations from a standard curve.

In other embodiments, absolute quantitation of the peptide(s) of interest can be determined. Quantitative measurements of the peptide(s) of interest are preferably performed by the use of internal standard peptide(s). Internal standard peptide(s) and optionally the peptide(s) of interest are for example differentially labeled in such a way that such internal standard(s) are structurally and chemically similar to the peptide(s) of interest, though the mass is different due to the label. Thus, the eluent from the separation process comprises an amount of the peptide(s) of interest that is in proportion to the known amount of those labeled internal standard peptide(s) in the sample, as prepared. It is then possible to relate the amount of the peptide(s) of interest in the sample to the amounts of the antibody of interest from which it originated via the standard curve. In a preferred embodiment, the internal standard peptide(s) are isotopically labeled for example deuterated, and incubated at a defined concentration with the antibody fragments after pepsin deactivation, for example.

As used herein, "isotopically labeled" refers to a peptide that has been enriched synthetically with one or more heavy atom isotopes (e.g. stable isotopes such as Deuterium, ¹³C, ¹⁵N, ¹⁸O, ³⁷Cl or ⁸¹Br).

The quantitative calibration curve is generated using a dilution series of the antibody of interest in the blank biological matrix, such as serum. Each sample is processed in the same manner as the actual test samples. The internal standard peptide(s) are added, at a known fixed amount to each of the calibration curve dilutions, just after the pepsin digestion has been deactivated just as with the test samples. Once the tryptic, or other specific protease, digestion is complete the sample will contain a known level of the internal standard peptide and some population of the peptide of interest that was produced from the digestion of the antibody of interest. The ratio of the signal intensities of the peptide(s) of interest to the corresponding internal standard peptide(s) signal intensities in the dilution series is used to create a calibration curve. This calibration curve data can then be applied to the test samples to quantify the antibody of interest because the procedure used and amount of internal standard is identical.

Quantitation of the peptide(s) of interest, based on the internal standard and calibration curve, thereby enables determination of the quantity of the antibody of interest that was originally present in the biological sample.

The following examples are intended for purposes of illustration only and should not be construed to limit the scope of the invention, as defined in the claims appended thereto.

### LEGENDS OF THE FIGURES

Figure 1: SDS-PAGE gel of human serum digested with varying amounts of porcine pepsin.
Figure 2: Representative HPLC-MS/MS chromatogram of human serum spiked with mAb X obtained during the abbreviated validation.
Figure 3: Representative peptide H-T4 standard curve demonstrating linearity and range.
Figure 4: mAb X concentration versus time: comparison of ELISA and HPLC-MS/MS data for mAb X administered i.v. in a rat.

### EXAMPLE

The example below describes in detail one specific embodiment of the method of the invention for quantitating an antibody of interest in a serum sample, which shall hereafter be referred to as mAb X.

### 1. Method Development

### Pepsin Depletion

### Evaluation

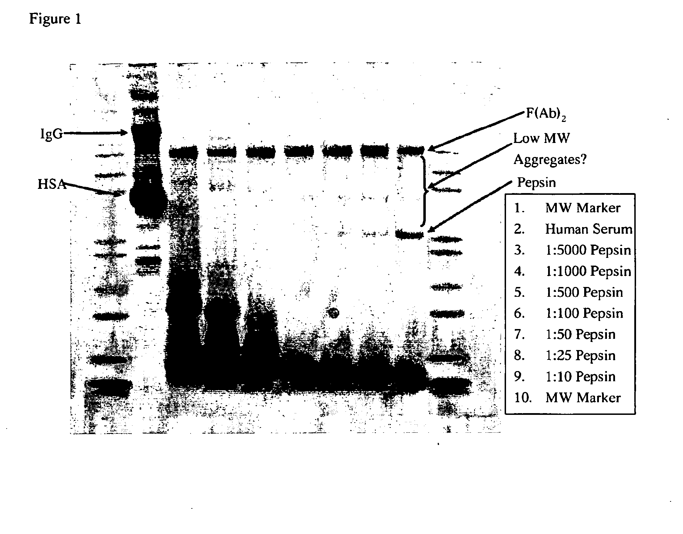

To evaluate the efficacy of pepsin to deplete human serum and selectively enrich for F(ab)₂ , 50 µL aliquots of commercially obtained pooled human serum (Equitech Bio) were adjusted to pH 3.5 with dilute hydrochloric acid and digested overnight with various concentrations of porcine pepsin (Roche Applied Science Cat. # 03 117 901 001) at 37°C assuming approximately 100 mg/mL total serum protein. Pepsin digests were run alongside untreated human serum on a Novex NUPAGE 4-12% gel (In Vitrogen) under non-reducing conditions using MES running buffer at 200V constant for 40 minutes, stained with coommassie blue (Bio-rad), destained and subsequently scanned using an Alpha Innotech Multilmage Light Cabinet. Figure 1 demonstrates that pepsin successfully depleted human serum while producing stable F(ab)₂s even at high concentrations of enzyme. Pepsin is suitable for use at concentrations ranging from 1:100 to 1:10 (wt/wt) enzyme:protein ratios. It may be possible to use higher concentrations although this was not evaluated.

### Optimized Protocol

### Instrumentation

The HPLC-MS/MS system consisted of an Agilent 1100 capillary LC system (capillary pump, autosampler and degasser), an Analytical Sales Hot Sleeve™ column heater and an Applied Biosystems 4000QTRAP mass spectrometer operating as a triple quadrupole equipped with nanoelectrospray source using a New Objective 50 micron ID metal TaperTip™ as the electrospray needle. The system was run using Analyst 1.4 software.

### Peptide Selection

The first step to determining if mAb X will be suitable to use in an HPLC-MS/MS-based pharmacokinetic assay will be to perform an analysis to identify the most unique residues in the variable light and variable heavy chain regions of the antibody. These can be found in the Fab portion of the molecule, making the pepsin-based depletion strategy feasible. This analysis is done *in silico* against human IgGs, When selecting the putative candidate for quantitation, ideally, the peptide should: have good ionization and chromatographic properties, no residues highly susceptible to modification (such as methionine) and no background in the total human IgGs. Based upon these criteria, one peptide, referred hereafter as H-T4 and corresponding to a peptide fragment in the heavy chain, was chosen. The stable isotope labeled internal standard (IS), with deuterated analogue of valine, was synthesized. The IS was spiked at a constant concentration into all samples and standards to normalize the signal. This adjusted for variations in sample handling and the analysis such as run-to-run and sample-to-sample sample preparation, ionization efficiencies and injection volumes.

### Mass Spectrometry Conditions

The IS was reconstituted in water to a concentration of 1 mM. It was further diluted to 0.5 nM in 30% acetonitrile, 0.1 % formic acid and directly infused into the mass spectrometer. The Quantitative Optimization tool in the Analyst 1.4 software was used to optimize the MRM conditions. Briefly, in MRM, the first quadrupole is used to isolate the parent mass of the peptide. The parent mass is representative of the overall sequence of the peptide. It then passes into the collision quadrupole where it is fragmented. The third quadrupole is used to monitor selected fragment ions. The fragments generated are indicative of the internal peptide sequence. Taken together Q1 and Q3 masses represent distinct transitions and, choosen appropriately, are highly specific to the sequence. In this case, the two most intense transitions after optimization were chosen by the software and were summed. Choosing multiple transitions increases the selectivity of the analysis. Table 1 shows the optimized MS conditions for one specific peptide of interest and the internal standard. Q1 and Q3 were operated at unit resolution.

**Table 1 Optimized MRM Parameters for H-T4 and IS Peptides.**

| Q1 mass | Q3 mass | Collision Energy | Collision Exit Potential | Dwell Time (msec) |
|---|---|---|---|---|
| 786.40 | 886.90 | 30 | 28 | 100 |
| 786.40 | 936.40 | 30 | 26 | 100 |
| 789.10 | 890.90 | 30 | 28 | 100 |
| 789.10 | 940.40 | 30 | 26 | 100 |
| | | | | |
| Curtain | 20 | | lonspray | 3250 |
| Gas | 10 | | (V) | 20 |
| EP | 0 | | GS1 | 200 |
| GS2 | 10 | | IHT | 50 |
| CAD | | | DP (V) | |

### Final HPLC conditions

The HPLC column was a 0.3 x 75 mm Zorbax C8 Stablebond300 and was heated to 60°C. The flow rate was set to 5 µL/min. Mobile phase A was composed of 0.1% formic acid in water and mobile phase B was 0.1 % formic acid in acetonitrile. A gradient was used to elute the peptide into the mass spectrometer. The first 12.5 minutes of the flow was diverted to waste, the flow switched inline for the subsequent 6 minutes followed by diverting the flow back to waste for the remainder of the analysis time to minimize the potential for contaminating the source.

**Table 2 HPLC Gradient Conditions.**

| Time (Min) | %Solvent B |
|---|---|
| 0.0 | 10.0 |
| 10.0 | 10.0 |
| 17.5 | 28.0 |
| 20.0 | 90.0 |
| 30.0 | 90.0 |
| 32.5 | 10.0 |
| 47.5 | 10.0 |

### Sample and Standard Treatment

For pharmacokinetic study and validation samples, 20-50 µL serum per sample was pipetted into a 96-well 2 mL plate. Samples were pH adjusted to 3.5 ± 0.25 by adding 333 mM hydrochloric acid to a final concentration of 67 mM. Pepsin (20 mg/mL in water) was subsequently added to a final concentration of 2 µg pepsin/µL serum and the plate was capped, centrifuged briefly, and vortexed. Digestion proceeded overnight at 37°C in an oven.

### Tryptic Digestion

Upon completion of pepsin digestion, samples were diluted to 475 µL with 2M urea, 1% β-octylglucopyranoside (OG), 10 mM DTT, 50 mM ammonium bicarbonate pH 7.8 and the plate was vortexed. Raising the pH above 6.0 irreversibly inactivates pepsin. Urea and OG act to denature the proteins and solubilize any precipitated proteins/low molecular weight aggregates formed as a function of the pepsin digestion. 2.5 pmoles IS was spiked into each sample. The plate was incubated at 37°C for two hours in an oven to facilitate disulfide bond reduction by DTT. IAA was added to a final concentration of 20 mM and the plate incubated at ambient temperature in the dark.for 1 hour to carboxamidomethylate free cysteine residues. Bovine trypsin (Sigma Cat. #T-1426) was prepared at 2.5 mg/mL in 1 mM hydrochloric acid and 0.5 µg trypsin/µL serum was added. The plate was capped, briefly centrifuged, and vortexed. Samples were digested overnight at 37°C in an oven. Formic acid was added the following day to a final concentration of 1% (v/v) to terminate the digestion.

### Solid Phase Extraction

Digests were cleaned-up by solid phase extraction (SPE) using Oasis MCX 96-well 30 µm, 30 mg plates (Waters Corp.) in a vacuum manifold. This removed excess salts, detergent, residual serum lipids, non-tryptic peptides resulting from pepsin digestion and insoluble materials which may have clogged the instrument, decreased LOQs or otherwise reduced the ruggedness and reproducibility of the assay. It also permitted significant concentration of the samples. The manufacturer's recommended protocol was followed with minor modifications. Briefly, Oasis MCX wells were prewashed with 1.0 mL methanol followed by 1.0 mL HPLC grade water. Samples were loaded and then washed with 2 x 1.0 mL 1% acetic acid followed by 2 x 1.0 mL methanol. Subsequently, 1.0 mL 5% ammonium hydroxide in 50% methanol, 45% ethanol was used to elute into 96-well 2 mL collection plates. Samples were evaporated to dryness and stored at ≤-50°C. Prior to HPLC-MS/MS analysis, the digests were freshly reconstituted in 1% trifluoracetic acid. 2.5 µL serum equivalent was injected onto the HPLC column.

### 2. Method Evaluation

### Abbreviated Validation

A limited validation in human serum of the HPLC-MS/MS method was performed in order to demonstrate suitable assay performance according to the accepted guidelines for ELISA-based approaches (DeSilva et. al. (2003) and Smolec et. al. (2005)). Human serum was collected from four healthy volunteer subjects, 2 females and 2 males. The antibody of interest, mAb X, was spiked into one serum sample at 250 µg/mL and into three others at 50 µg/mL. Spiked samples of two of the 50 µg/mL samples were serially diluted with corresponding serum to 5.0 µg/mL and 0.5 µg/mL, respectively. Samples were prepared in triplicate and analyzed on three assay occasions using 50 µL aliquots per replicate. The standard curves were prepared by serially diluting a commercially obtained human serum pool (Equitech Bio) from 500 to 0.1 µg/mL and performing the entire procedure (3 replicates/point).

### Rat Pharmacokinetic Study - Comparison to ELISA

A comparison with ELISA was obtained by analyzing samples from a rat dosed with mAb X at 10 mg/kg with the HPLC-MS/MS method. The ELISA was performed using a standard protocol with an anti-Human Fc antibody for capture followed by an anti-human Fab antibody for detection to determine the pharmacokinetics of mAb X in a rat. For the MS analysis, due to limited sample volume, serum from each time point was prepared in singlet (20 µL serum) and analyzed in triplicate. The standard curve was prepared by serially diluting mAb X with a commercially-obtained rat serum pool (Sigma-Aldrich) from 500 to 0.5 µg/mL of mAb X and performing the entire sample preparation procedure (3 replicates/point). In addition, rat serum spikes of mAb X at 50 µg/mL, 5 µg/mL and 0.5 µg/mL were analyzed to determine accuracy in this matrix.

### Results

### Abbreviated Validation

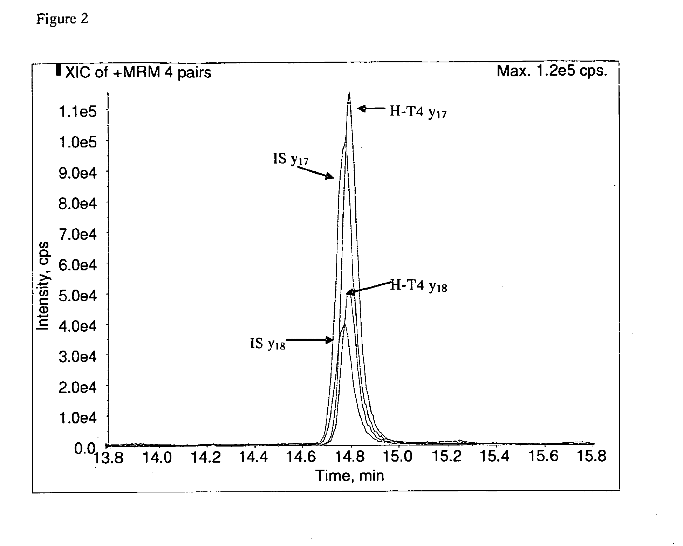

Figure 2 is a representative antibody spike HPLC-MS/MS chromatogram obtained during the abbreviated validation demonstrating co-elution of the IS with the peptide of interest (called hereafter in the specific example H-T4).

### Linearity and Range

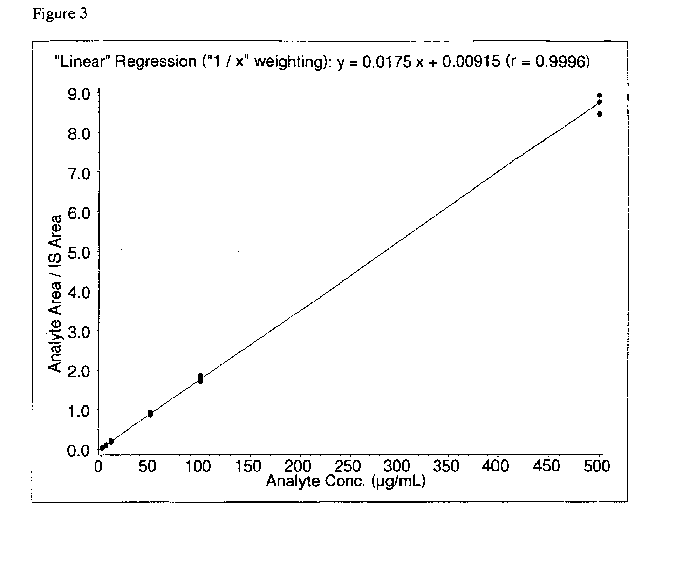

Linear dynamic range is evaluated as the ability of the method to produce a signal that is directly proportional to the concentration over a given range. Range is the interval between the upper and lower levels of analyte that have been demonstrated to be determined with suitable precision and accuracy. Using linear through zero regression with 1/x weighting the MRM method for mAb X was linear from 0.5 to 500 µg/mL with R2 ≥ 0.999 for all three assays (Figure 3). This is a three order of magnitude dynamic range and is consistent with that reported in the literature for MRM. It should be noted that concentrations above 500 µg/mL were not evaluated in this study. It may be possible to extend the range beyond the current estimate. The QTRAP is reported to be capable of 3.5 to 4 orders of magnitude linear dynamic range.

### Precision and Accuracy

The method's accuracy is a measurement of closeness of agreement of the measured value to the true or accepted value. Over the range 0.5 to 250 µg/mL, the percent accuracy (or percent recovery) of spikes across the three assays was within 10% of the theoretical.

Precision is a measurement of the reproducibility of the method at any given concentration. Precision is often expressed as the coefficient of variation (CV) of replicate measurements and is usually evaluated both within and between assays. The within and between day CVs for all concentrations were less than 7%.

### Limit of Quantitation

The limit of quantitation (LOQ) is defined to be that concentration at which quantitative results can be reported with a high degree of confidence and for which the signal: noise (S/N) is at least 10:1. Given these criteria, the S/N was calculated for the 0.5 µg/mL spike level by determining the absolute signal intensity for the H-T4 peak and comparing it to the intensity of the noise in the one minute region following the peak. The S/N was 34.4 ±8.8 across all three assays. The LOQ should be 0.25 µg/mL which is adequate to calculate half life for most common preclinical dosing regimes.

### Selectivity (Specificity)

The specificity of an assay is its ability to assess unequivocally the analyte in the presence of components which may be expected to be present. Typically these might include impurities, degradants, and matrix components. To assess the specificity of the HPLC-MS/MS method, blank serum samples from each of the four subjects and the commercially obtained serum pool were analyzed. The H-T4 of mAb X was not detected in any of the blanks in any of the assay occasions

### Rat Pharmacokinetic Study - Comparison to ELISA

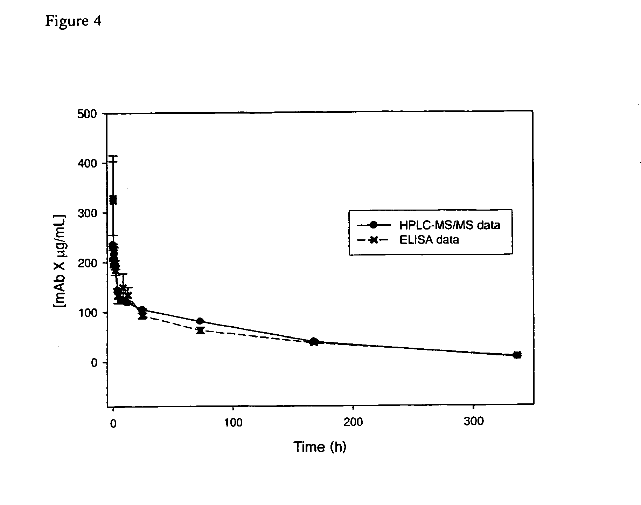

Figure 4 represents a comparison of the HPLC-MS/MS method with ELISA. Very close agreement was observed between the methods. In addition, rat serum spikes were within 6% of the theoretical values thus reinforcing the accuracy of the HPLC-MS/MS method obtained during the abbreviated validation.

### REFERENCES

Anderson L, Hunter CL (2006) Quantitative mass spectrometric multiple reaction monitoring assays for major plasma proteins. Mol Cell Proteomics; 5(4):573-588.
DeSilva B, Smith W, Weiner R, Kelley M, Smolec J, Lee B, Khan M, Tacey R, Hill H, Celniker A (2003) Recommendations for the bioanalytical method validation of ligand-binding assays to support pharmacokinetic assessments of macromolecules. Pharm Res; 20(11):1885-1900.
Gerber SA, Rush J, Stemman O, Kirschner MW, Gygi SP (2003) Absolute quantification of proteins and phosphoproteins from cell lysates by tandem MS. Proc Natl Acad Sci; 100(12):6940-6945.
Huston, J.S., Levinson, D., Mudgett, H.M., Tai, M.S., Novotny, J., Margolies, M.N., et al. (1988) Protein engineering of antibody binding sites: recovery of specific activity in an anti-digoxin single-chain Fv analogue produced in Escherichia coli. Proc Natl Acad Sci; 85: 5879-5883.
Jones RG, Landon J (2003) A protocol for 'enhanced pepsin digestion': a step by step method for obtaining pure antibody fragments in high yield from serum. J Immunol Methods; 275(1-2):239-250.
Jones RG, Landon J (2002) Enhanced pepsin digestion: a novel process for purifying antibody F(ab')(2) fragments in high yield from serum. J Immunol Methods; 263(1-2):57-74.
Rousseaux J, Biserte G, Bazin H (1980) The differential enzyme sensitivity of rat immunoglobulin G subclasses to papain and pepsin. Mol Immunol; 17(4):469-482.
Smith, TF and Waterman, MS (1981) Overlapping Genes and Information-Theory J Theoret Biol, 91, 379-380.
Smolec J, DeSilva B, Smith W, Weiner R, Kelly M, Lee B, Khan M, Tacey R, Hill H, Celniker A, Shah V, Bowsher R, Mire-Sluis A, Findlay JW, Saltarelli M, Quarmby V, Lansky D, Dillard R, Ullmann M, Keller S, Karnes HT (2005) Bioanalytical method validation for macromolecules in support of pharmacokinetic studies. Pharm Res; 22(9):1425-1431.
Ward ES, Gussow D, Griffiths AD, Jones PT, Winter G. (1989) Binding activities of a repertoire of single immunoglobulin variable domains secreted from Escherichia coli. Nature. Oct 12;341(6242):544-6.

## Claims

1. An in vitro method for determining the amount of an antibody of interest comprised in a biological sample, said biological sample further comprising proteins other than antibodies, **characterized in that** said method comprises the steps of: a) incubating the biological sample with a protease capable of digesting non-immunoglobin proteins, but retaining at least the variable region of antibodies under conditions appropriate for proteolysis of the non-immunoglobulin proteins and digestion of the antibody molecules to produce antibody fragments comprising at least a CDR region; and, b) determining the amount of the antibody fragments derived from the antibody of interest; said amount of antibody fragments correlating with the amount of antibody of interest in the biological sample.

2. The method of Claim 1, wherein said protease in step a) is selected among the group of proteases capable of producing Fab or F(ab)2 fragments.

3. The method of Claim 2, wherein said protease in step a) is pepsin or a suitable homologue capable of producing stable F(ab)2 fragments.

4. The method of Claim 1, 2 or 3, wherein step b) comprises the following sub-steps of: b1) addition of one or more internal standard peptides to the antibody fragments generated in step a), after deactivation of pepsin; b2) digesting the sample of step b1) with another protease capable of digesting non-immunoglobin proteins, without being capable of retaining at least the variable region of antibodies, and optionally, extracting the sample peptides ; b3) partially separating the extracted peptides from one another in one or more samples; b4) measuring by mass spectrometry the mass or mass to charge ratio of the sample peptides, along with selected fragment ions thereof, present in one or more of the separated samples and identifying peak signals corresponding to one or more peptides of interest from the antibody of interest as well as the internal standard peptides; and, b5) determining the amount of the peptides of interest in the sample, thereby determining the amount of the antibody of interest comprised in the biological sample.

5. The method of Claim 4, wherein step b5) consists of comparing said peak signals to peak signals derived from a standard curve of antibody added at varying known concentrations into blank biological source material using the internal standard peptides for signal normalization for determining the amount of the peptides of interest in the sample, thereby determining the amount of the antibody of interest comprised in the biological sample.

6. The method of Claim 4 or 5, wherein the separation step b3) is performed by separating the sample peptides by chromatographic separation.

7. The method of Claim 4, 5 or 6, wherein the mass spectrometry analysis step b4) is performed by tandem mass spectrometry (MS/MS).

8. The method of any of Claims 4-7, wherein said internal standard peptides are isotopically labeled peptides.

9. The method of any of Claims 4 to 8, wherein said peptides of interest comprise at least a portion selected in the variable region of the antibody of interest, appropriate for discriminating between the peak signals corresponding specifically to the fragments of the antibody of interest and non specific signal corresponding to other protein fragments.

10. The method of Claim 9, wherein said peptides of interest comprise at least a portion selected among those which comprise unique amino acid residues in the variable light and/or heavy chain regions of the antibody when compared to corresponding native IgGs amino acid residues obtained from a native IgGs sequence database.

11. The method of any of Claims 4-10, wherein said mass spectrometry analysis is performed using a triple quadrupole mass spectrometer.

12. The method of any of Claims 1-11, wherein said biological sample is a serum, plasma, tissue or cell line derived from any species.

13. The method of any of Claims 1-12, wherein the antibody of interest is a non-naturally occurring antibody to be administered to a mammal.

14. The method of Claim 13, wherein said antibody of interest is a potential therapeutic product.

15. The method of any of Claims 1-14, wherein said method does not have any step of antibody enrichment by affinity columns or albumin depletion.

## Patentansprüche

1. In-vitro-verfahren zur Bestimmung der in einer biologischen Probe enthaltenen Menge eines interessierenden Antikörpers, wobei die biologische Probe ferner andere Proteine als Antikörper enthält, **dadurch gekennzeichnet, dass** das Verfahren die folgenden Schritte umfasst: a) Inkubieren der biologischen Probe mit einer zur Verdauung von Nicht-Immunglobulin-Proteinen fähigen Protease, wobei jedoch wenigstens der variable Bereich von Antikörpern erhalten bleibt, unter für die Proteolyse der Nicht-Immunglobulin-Proteine und Verdauung der Antikörpermoleküle unter Erhalt von wenigstens einen CDR-Bereich umfassenden Antikörperfragmenten geeigneten Bedingungen; und b) Bestimmen der Menge der von dem interessierenden Antikörper stammenden Antikörperfragmente, wobei die Menge an Antikörperfragmenten mit der Menge an interessierendem Antikörper in der biologischen Probe korreliert.

2. Verfahren nach Anspruch 1, wobei die Protease in Schritt a) unter der Gruppe von zur Produktion von Fab- oder F(ab)₂-Fragmenten fähigen Proteasen ausgewählt ist.

3. Verfahren nach Anspruch 2, wobei es sich bei der Protease in Schritt a) um Pepsin oder ein geeignetes, zur Produktion stabiler F(ab)₂-Fragmente fähigen Homolog handelt.

4. Verfahren nach Anspruch 1, 2 oder 3, wobei Schritt b) die folgenden Teilschritte umfasst: b1) Zugabe eines oder mehrerer Innerer-Standard-Peptide zu den in Schritt a) erzeugten Antikörperfragmenten, und zwar nach Deaktivierung von Pepsin; b2) Verdauen der Probe aus Schritt b1) mit einer anderen, zur Verdauung von Nicht-Immunglobulin" Proteinen, jedoch nicht zur Erhaltung wenigstens des variable Bereichs von Antikörpern fähigen Protease und gegebenenfalls Extrahieren der Probenpeptide; b3) teilweises Trennen der extrahierten Peptide voneinander in eine oder mehrere Proben; b4) massenspektrometrisches Messen der Masse oder des Masse-zu-Ladung-Verhältnisses der Probenpeptide zusammen mit ausgewählten Fragmentionen davon, vorliegend in einer oder mehreren der getrennten Proben, und Identifizieren von einem oder mehreren interessierenden Peptiden aus dem interessierenden Antikörper ebenso wie den Innerer-Standard-Peptiden entsprechenden Signalspitzen; und b5) Bestimmen der Menge der interessierenden Peptide in der Probe, wodurch die in der biologischen Probe enthaltene Menge des interessierenden Antikörpers bestimmt wird.

5. Verfahren nach Anspruch 4, wobei Schritt b5) darin besteht, dass man zur Bestimmung der Menge der interessierenden Peptide in der Probe die Signalspitzen mit aus einer Standardkurve von in verschiedenen bekannten Konzentrationen zu Leermaterial der biologischen Quelle gegebenem Antikörper gewonnenen Signalspitzen vergleicht, wobei zur Signalnormierung die Innerer-Standard-Peptide verwendet werden, wodurch die in der biologischen Probe enthaltene Menge des interessierenden Antikörpers bestimmt wird.

6. Verfahren nach Anspruch 4 oder 5, wobei der Trennschritt b3) mittels Probenpeptidtrennung durch chromatographische Trennung erfolgt.

7. Verfahren nach Anspruch 4, 5 oder 6, wobei der massenspektrometrische Analyseschritt b4) mittels Tandem-Massenspektrometrie (MS/MS) erfolgt.

8. Verfahren nach einem der Ansprüche 4-7, wobei es sich bei den Innerer,-Standard-Peptiden um isotopenmarkierte Peptide handelt.

9. verfahren nach einem der Ansprüche 4 bis 8, wobei die interessierenden Peptide wenigstens einen in dem variablen Bereich des interessierenden Antikörpers ausgewählten Anteil umfassen, der sich zur Unterscheidung zwischen den spezifisch den Fragmenten des interessierenden Antikörpers entsprechenden Signalspitzen und dem anderen Proteinfragmenten entsprechenden nicht spezifischen Signal eignet.

10. Verfahren nach Anspruch 9, wobei die interessierenden Peptide wenigstens einen unter solchen, die einmalige Aminosaurereste in den variablen Bereichen der leichten und/oder schweren Kette des Antikörpers im Vergleich mit entsprechenden, aus einer Sequenzdatenbank nativer IgGs erhaltenen Aminosäureresten native IgGs umfassen, ausgewählten Anteil umfassen.

11. Verfahren nach einem der Ansprüche 4-10, wobei die massenspektrometrische Analyse unter Verwendung eines Tripel-Quadrupol-Massenspektrometers erfolgt.

12. Verfahren nach einem der Ansprüche 1-11, wobei es sich bei der biologischen Probe um ein Serum, Plasma, Gewebe oder eine Zelllinie handelt, das bzw. die aus einer beliebigen Spezies gewonnen wurde.

13. Verfahren nach einem der Ansprüche 1-12, wobei es sich bei dem interessierenden Antikörper um einen einem Säuger zu verabreichenden nicht natürlich vorkommenden Antikörper handelt.

14. Verfahren nach Anspruch 13, wobei es sich bei dem interessierenden Antikörper um ein potentielles therapeutisches Produkt handelt.

15. Verfahren nach einem der Ansprüche 1-14, wobei das Verfahren keinen Schritt der Antikörperanreicherung mittels Affinitätssäule oder Albuminabreicherung aufweist.

## Revendications

1. Procédé *in vitro* pour déterminer la quantité d'un anticorps d'intérêt présent dans un échantillon biologique, ledit échantillon biologique comprenant en outre des protéines autres que des anticorps, **caractérisé en ce que** ledit procédé comprend les étapes suivantes : a) incubation de l'échantillon biologique avec une protéase capable de digérer des protéines non immunoglobulines, mais conservant au moins une région variable d'anticorps dans des conditions convenant à la protéolyse des protéines non immunoglobulines et à la digestion des molécules d'anticorps pour produire des fragments d'anticorps comprenant au moins une région CDR ; et b) détermination de la quantité des fragments d'anticorps qui dérivent de l'anticorps d'intérêt ; ladite quantité de fragments d'anticorps ayant une corrélation avec la quantité de l'anticorps d'intérêt dans l'échantillon biologique,

2. Procédé de la revendication 1, dans lequel ladite protéase de l'étape a) est choisie dans le groupe des protéases capables de produire des fragments Fab ou F(ab)₂.

3. Procédé de la revendication 2, dans lequel ladite protéase de l'étape a) est la pepsine ou un homologues approprié, capable de produire des fragments F(ab)₂ stables.

4. Procédé de la revendication 1, 2 ou 3, dans lequel l'étape b) comprend les sous-étapes suivantes : b1) addition d'un ou plusieurs peptides étalons internes aux fragments d'anticorps produits dans l'étape a), après désactivation de la pepsine ; b2) digestion de l'échantillon de l'étape b1) avec une autre protéase capable de digérer des protéines non immunoglobulines, sans être capable de conserver au moins la région variable d'anticorps, et en option extraction des peptides échantillons ; b3) séparation partielle, les uns des autres, des peptides extraits dans un ou plusieurs échantillons ; b4) mesure par spectrométrie de masse de la masse ou du rapport masse/charge des peptides échantillons, en même temps que de leurs ions fragments sélectionnés, présents dans un ou plusieurs des échantillons séparés, et identification des signaux de crête correspondant à un ou plusieurs peptides d'intérêt à partir de l'anticorps d'intérêt, ainsi qu'à des peptides étalons internes ; et b5) détermination de la quantité des peptides d'intérêt dans l'échantillon, de façon à déterminer la quantité de l'anticorps d'intérêt qui se trouve dans l'échantillon biologique.

5. Procédé de la revendication 4, dans lequel l'étape b5) consiste en une comparaison des signaux de crête à des signaux de crête qui dérivent d'une courbe étalon d'anticorps ajoutées à différentes concentrations connues à un matériau source biologique blanc, par utilisation des peptides étalons internes pour la normalisation des signaux dans le but de déterminer la quantité des peptides d'intérêt dans l'échantillon, pour déterminer de ce fait la quantité de l'anticorps d'intérêt qui se trouve dans l'échantillon biologique.

6. Procédé de la revendication 4 ou 5, dans lequel l'étape de séparation b3) est mise en oeuvre par séparation des peptides échantillons par séparation chromatographique.

7. Procédé de la revendication 4, 5 ou 6, dans lequel l'analyse par spectrométrie de masse de l'étape b4) est mise en oeuvre par une spectrométrie de masse en tandem (SM/SM).

8. Procédé de l'une quelconque des revendications 4 à 7, dans lequel lesdits peptides étalons internes sont des peptides à marquage isotopique.

9. Procédé de l'une quelconque des revendications 4 à 8, dans lequel lesdits peptides d'intérêt comprennent au moins une partie choisie dans la région variable de l'anticorps d'intérêt, permettant une discrimination entre les signaux de crête correspondant d'une manière spécifique aux fragments de l'anticorps d'intérêt, et le signal non spécifique correspondant à d'autres fragments protéiques.

10. Procédé de la revendication 9, dans lequel lesdits peptides d'intérêt comprennent au moins une partie choisie parmi celles qui comprennent des résidus d'acides aminés uniques dans les régions variables de la chaîne légère et/ou de la chaîne lourde de l'anticorps par comparaison avec des résidus d'acides aminés IgG natifs correspondants obtenus à partir d'une base de données de séquences d'IgG natifs.

11. Procédé de l'une quelconque des revendications 4 à 10, dans lequel ladite analyse par spectrométrie de masse est mise en oeuvre par utilisation d'un spectromètre de masse à trois quadripôles.

12. Procédé de l'une quelconque des revendications 1 à 11, dans lequel ledit échantillon biologique est un sérum, un plasma, un tissu ou une lignée cellulaire dérivant d'une espèce quelconque.

13. Procédé de l'une quelconque des revendications 1 à 12, dans lequel l'anticorps d'intérêt est un anticorps non naturel, destiné à être administré à un animal.

14. Procédé de la revendication 13, dans lequel ledit anticorps d'intérêt est un produit thérapeutique potentiel.

15. Procédé de l'une quelconque des revendications 1 à 14, lendit procédé ne comportant aucune étape d'enrichissement de l'anticorps par colonnes d'affinité ou épuisement de l'albumine.
